# EUROPÄISCHE PATENTSCHRIFT

(11) **EP 0 659 723 B1**
(45) Veröffentlichungstag und Bekanntmachung des Hinweises auf die Patenterteilung: **23.07.1997**
(21) Anmeldenummer: 94119302.1
(22) Anmeldetag: 07.12.1994
(51) Int. Cl.: C07C 43/04, C07C 41/06, B01J 31/10

(54) **Verfahren zur Herstellung von Alkyl-tert.-alkyl-ether enthaltenden Kohlenwasserstoffgemischen**
Process for the preparation of hydrocarbon mixtures containing alkyl tert-alkyl ethers
Procédé pour la préparation de mélanges d'hydrocarbures contenant des ethers alkyl tertio-alkyls

(30) Priorität: 20.12.1993 DE 4343453
(43) Veröffentlichungstag der Anmeldung: 28.06.1995
(73) Patentinhaber: BAYER AG, 51368 Leverkusen (DE); EC ERDÖLCHEMIE GMBH, D-50769 Köln (DE)
(72) Erfinder: Bister, Dr. Hans Jürgen, D-40479 Düsseldorf (DE); Stüwe, Dr. Arnd, D-51373 Leverkusen (DE); Tschorn, Dipl.-Ing. Herbert, D-41539 Dormagen (DE); Wagner, Dr. Rudolf, D-51061 Köln (DE); Strüver, Dr. Werner, D-51375 Leverkusen (DE)

(56) Entgegenhaltungen:
- EP-A- 0 043 986
- EP-A- 0 048 893
- EP-A- 0 197 348
- EP-A- 0 338 309
- EP-A- 0 371 692

## Beschreibung

Die vorliegende Erfindung betrifft ein Verfahren zur Herstellung von Kohlenwasserstoffgemischen mit einem Gehalt an Alkyl-tert.-alkyl-ether oder Alkylen-bis-(tert.-alkyl-ether) durch katalysierte Umsetzung eines rohen Kohlenwasserstoffgemisches mit niederen Alkanolen oder Alkandiolen an einem Katalysator. Als rohe Kohlenwasserstoffgemische können solche mit einem Schwefelgehalt bis zu 1 500 ppm, von dem bis zu 500 ppm in Form von Mercaptanen, Disulfiden oder einem Gemisch von beiden vorliegen, eingesetzt werden. Als schwefelresistentes katalytisches Mittel wird hierbei ein Ionenaustauscher mit SO₃H-Gruppen eingesetzt, der mit einem Gemisch von einem Metall aus einer ersten Gruppe von Pd, Ru, Rh und Pt mit einem Metall aus einer zweiten Gruppe von Fe, Co, Ni, Cu, Ag und W belegt ist.

Die Erfindung betrifft auch dieses katalytische Mittel.

Im erfindungsgemäßen Verfahren wird weiterhin durch gleichzeitigen Zusatz von Wasserstoff der Gehalt an hoch ungesättigten Verbindungen weitgehend eliminiert. Weiterhin erfolgt im erfindungsgemäßen Verfahren eine Isomerisierung der endständigen Monoolefine. Alle diese durch das katalytische Mittel bewirkten Umsetzungen werden durch den Schwefelgehalt des rohen Kohlenwasserstoffgemisches nur in wesentlich verringertem Maße beeinträchtigt.

Es ist seit langem bekannt, Leichtbenzine und Naphtha rein thermisch oder katalytisch zu cracken, wobei man in gewünschter Weise Ethylen und Propylen erhält; diese beiden Olefine werden hauptsächlich zu den entsprechenden Polymeren weiterverarbeitet. Daneben enthalten solche Crack-Produkte weitere Alkene, die geradkettig oder verzweigt sein können, Alkane und Aromaten. Zu deren Aufarbeitung wird das nach der Abtrennung von Ethylen und Propylen verbleibende Gemisch in Destillationsschnitte aufgeteilt, die im allgemeinen den Bereich gleicher Kohlenstoffzahl oder benachbarten Kohlenstoffzahlen umfassen. Die in diesen Destillationsschnitten vorhandenen tert.-Alkene können an sauren Katalysatoren mit niederen Alkanolen zu Alkyl-tert.-alkyl-ethern umgesetzt werden. Diese Ether können isoliert werden und stellen begehrte Lösungsmittel und Octanzahlverbesserer in Vergaserkraftstoffen dar.

Es ist jedoch auch möglich, solche veretherten tert.-Alkene im Kohlenwasserstoffgemisch zu belassen und ein solches Gemisch ohne weitere Aufarbeitung dem Vergasertreibstoff als Octanzahlverbesserer zuzusetzen. In einem solchen Fall ist es wünschenswert, sogenannte Gum-Bildner zu eliminieren. Unter Gum wird im Zusammenhang mit Kraftstoffen ein Gehalt an oligomeren oder polymeren Stoffen im Kraftstoff verstanden, der bei der Analytik der Kraftstoffe als Abdampfrückstand zutage tritt. Ein Gehalt an Gum im Kraftstoff führt zu Verkokungen und Ablagerungen im Brennraum des Motors und ist daher unerwünscht. Gum-Bildner sind hoch ungesättigte Verbindungen, beispielsweise Diolefine und/oder Acetylen-Verbindungen. Gemäß EP 197 348 B1 gelingt die simultane Veretherung der tert.-Alkene und die Entfernung der Gum-Bildner durch ihre Umwandlung in Alkene dadurch, daß man entsprechende Destillationsschnitte von solchen rohen Kohlenwasserstoffen gleichzeitig mit Methanol und Wasserstoff an einem Katalysator behandelt, der aus einem handelsüblichen Kationenaustauscher (Styrol-Divinyl-benzol-Copolymer mit Sulfonsäuregruppen) in der H⁺-Form besteht, der mit Palladium belegt worden ist.

Es wurde weiterhin gefunden (EP 338 309 A), daß es möglich ist, an den beschriebenen Ionenaustauscher/Pd-Katalysatoren zusätzlich eine teilweise Isomerisierung von Alkenen vorzunehmen. So wird beispielsweise in den jeweiligen Destillationsschnitten Buten-1 in das für Alkylierungen gewünschte Buten-2 umgelagert; in den Destillationsschnitten mit höherer Kohlenstoffzahl wird insbesondere eine Isomerisierung verzweigter Olefine in der Art beobachtet, daß solche, deren Doppelbindung nicht am Verzweigungskohlenstoffatom steht, in solche umgewandelt werden, die dann tert.-Alkene darstellen und so zu einer weitergehenden Veretherung beitragen.

Es wurde nun beobachtet, daß die Hydrieraktivität zur Beseitigung der Gum-Bildner, insbesondere aber die wünschenswerte Isomerisierungsaktivität von Katalysatoren des Kationenaustauscher/Platinmetall-Typs, insbesondere solche des Kationenaustauscher/Pd-Typs unter dem Einfluß eines hohen Schwefelgehaltes im rohen Kohlenwasserstoffgemisch frühzeitig nachläßt und auf einen industriell nicht mehr verwertbaren Stand absinkt. Zur Beseitigung einer solchen unerwünschten Einflußnahme eines Schwefelgehaltes auf katalytische Umsetzungen ist es bisher erforderlich, eine separate Vorbehandlung der rohen Kohlenwasserstoffe an einem anorganischen, schwefelfesten Kontakt vorzunehmen oder zur katalytischen Umsetzung einen anderen schwefelfesten Katalysator einzusetzen, der jedoch nicht mehr die gewünschten mehrfachen Funktionen wie der Kationenaustauscher/Platinmetall-Typ ausüben kann. Eine weitere Möglichkeit besteht darin, das schwefelhaltige Feed zuvor mit einem "Scavenger", beispielsweise einem basische Gruppen tragenden Harz (Anionenaustauscher) zu behandeln. Diese Möglichkeit verursacht Kosten wie alle zusätzlichen Behandlungen, ist aber nur für ausreichend saure S-Verbindungen geeignet: Mercaptan-Verbindungen werden beispielsweise gebunden, nicht jedoch Thioether oder Disulfide.

Es war daher wünschenswert, ein Verfahren und geeignete katalytische Mittel hierzu zu finden, die schwefelfest sind und dennoch die beschriebenen mehrfachen Funktionen ausüben können, wobei gleichzeitig eine umständliche und kostenintensive separate Schwefelentfernung entfallen sollte.

Es wurde gefunden, daß eine solche Behandlung schwefelhaltiger roher Kohlenwasserstoffgemische möglich ist, wenn man katalytische Mittel auf der Basis eines Kationenaustauschers mit SO₃H-Gruppen einsetzt, der die weiter unten beschriebenen Metalle in den angegebenen Mengen enthält.

Die Erfindung betrifft daher ein Verfahren zur Herstellung eines Gemisches von Alkanen und Alkenen mit 4-8 C-Atomen und C₁-C₈-Alkyl-tert.-C₄-C₈-alkyl-ethern oder C₂-C₈-Alkylen-bis(C₄-C₈-tert.-alkyl-ethern), das weitgehend frei ist von hoch ungesättigten Verbindungen und das überschüssiges C₁-C₈-Alkanol oder C₂-C₈-Alkandiol enthalten kann, durch Umsetzung von
a) einem rohen Kohlenwasserstoffgemisch im Bereich von 4-8 C-Atomen, das C₄-C₈-tert.-Alkene neben anderen Alkenen und Alkanen enthält und hoch ungesättigte Verbindungen enthält, mit
b) einem C₁-C₈-Alkanol oder einem C₂-C₈-Alkandiol in einer Menge von 0,7-4 , bevorzugt 0,8-2,5, besonders bevorzugt 1-2 OH-Äquivalenten, bezogen auf die Molmenge an C₄-C₈-tert.-Alken, und gleichzeitig mit
c) Wasserstoff in einer Menge von 80-500, bevorzugt 100-200, % der zur hydrierenden Umwandlung der hoch ungesättigten Verbindungen in Alkene benötigten Menge
an einem makroporösen oder gelförmigen sauren, SO₃H-Gruppen enthaltenden Kationenaustauscher mit einem Vernetzungsgrad von 2-65 % und einer spezifischen Oberfläche von 5-750 m²/g trockenen Kationenaustauschers, der mit Metallen belegt worden ist, als katalytisches Mittel, wobei gleichzeitig die Veretherung der tert.-Alkene mit den Alkanolen, die Umwandlung der hoch ungesättigten Verbindungen und eine teilweise Isomerisierung der Alkene durchgeführt werden, das dadurch gekennzeichnet ist, daß ein rohes Kohlenwasserstoffgemisch mit einem gesamten Schwefelgehalt von 10-1500 ppm, von dem 5-500 ppm in Form von Mercaptanen, Disulfiden oder einem Gemisch beider vorliegen, eingesetzt wird und der Kationenaustauscher einen Gehalt von 0,2-20 g/ l des Kationenaustauschers, gerechnet als Metall, an einem Gemisch von einem Metall aus einer ersten Gruppe von Pd, Ru, Rh und Pt mit einem Metall aus einer zweiten Gruppe von Fe, Co, Ni, Cu, Ag und W aufweist, wobei die Metalle der ersten und zweiten Gruppe im Gewichtsverhältnis von 1:4-4:1, bevorzugt 1:2-2:1 zueinander stehen.

Die Erfindung betrifft weiterhin das katalytische Mittel zur Durchführung des obigen Verfahrens, bestehend aus einem makroporösen oder gelförmigen Kationenaustauscher mit SO₃H-Gruppen mit einem Vernetzungsgrad von 2-65 % und einer spezifischen Oberfläche von 5-750 m²/g trockenen Kationenaustauschers, der mit 0,2-20 g/l Kationenaustauscher, gerechnet als Metall, an einem Gemisch von einem Metall aus einer ersten Gruppe von Pd, Ru, Rh und Pt mit einem Metall aus einer zweiten Gruppe von Fe, Co, Ni, Cu, Ag und W aufweist, wobei die Metalle der ersten und zweiten Gruppe im Gewichtsverhältnis von 1:4-4:1, bevorzugt 1:2-2:1 zueinander stehen.

Die erfindungsgemäß einzusetzenden rohen Kohlenwasserstoffe enthalten tert.-Olefine, die einer Veretherung zugänglich sind, wie i-Buten, 2-Methyl-buten-1, 2-Methyl-penten-1 und weitere innerhalb des Bereiches von 4-8 C-Atomen, bevorzugt im Bereich von 4-6 C-Atomen. Die einzusetzenden rohen Kohlenwasserstoffgemische enthalten weiterhin verzweigte Alkene, bei denen die Doppelbindung nicht am Verzweigungskohlenstoffatom steht, die jedoch einer Isomerisierung zum entsprechenden tert.-Alken zugänglich sind, beispielsweise 3-Methyl-buten-1 und entsprechende im Bereich von 5-8, bevorzugt 5-6 C-Atomen.

Weiterhin enthalten die einzusetzenden rohen Kohlenwasserstoffgemische geradkettige und/oder verzweigte und/oder cyclische gesättigte und einfach ungesättigte Kohlenwasserstoffe mit im wesentlichen 4-8, bevorzugt 4-6 C-Atomen und geringe Mengen der anschließenden Homologen von allen genannten Kohlenwasserstoffarten, beispielsweise in einem C₅-Destillationsschnitt geringe Mengen an C₄- und C₆-Kohlenwasserstoffen.

Als Gum-Bildner enthalten die rohen Kohlenwasserstoffgemische Diolefine und/oder Acetylen-Verbindungen, die im Siedebereich der entsprechenden Destillationsschnitte liegen und hauptsächlich auch im Bereich der entsprechenden Zahlen an C-Atomen liegen.

Solche Kohlenwasserstoffgemische mit verschiedenen C-Zahlen und unterschiedlichem Grad an Ungesättigtheit sowie dem Gehalt an tert.-Olefinen stehen in petrochemischen Anlagen oder Raffinerien zur Verfügung und können beispielsweise durch Umsetzung von Naphtha, Liquid Petroleum Gas (LPG), Rohöldestillaten, Gasöl, Erdgaskondensaten oder anderen Ausgangskohlenwasserstoffgemischen in Steamcrackern, katalytischern Crackern (FCC), Isomerisierungs- oder Dehydrierungsanlagen gewonnen werden. Da viele der genannten Ausgangsstoffe einen Gehalt an Schwefel aufweisen, zeigen auch die Crack-Produkte und die daraus hervorgegangenen Destillationsschnitte einen solchen Gehalt an Schwefel. Er beträgt 10-1500 ppm, bevorzugt 20-1000 ppm, besonders bevorzugt 30-500 ppm, an Gesamtschwefel, von dem 5-500 ppm, bevorzugt 10-400 ppm, besonders bevorzugt 15-200 ppm, in Form von Mercaptanen, Disulfiden oder einem Gemisch beider vorliegen; diese Bindungsart des Schwefels zeigt besonders starke Desaktivierung von edelmetallhaltigen Katalysatoren. Der Rest des Schwefelgehaltes bis zu der genannten Obergrenze liegt vielfach in Form von Thiophenen vor, die edelmetallhaltige Katalysatoren weniger stark desaktivieren. Schwefelgehalte treten besonders häufig bei FCC-Produkten auf.

Als Beispiel für die Zusammensetzung solcher erfindungsgemäß einzusetzender roher Kohlenwasserstoffgemische sei die Zusammensetzung eines C₅-Destillationsschnittes aus einem katalytischen Cracker wie folgt angegeben:

| Stoff, Gew.-% (ca.) | Cat. Cracker (FCC) |
|---|---|
| C₄/Leichte | 1 - 5 |
| n-/i-Pentan | 30 - 35 |
| n-Penten | 25 - 30 |
| 3-Methyl-buten-1 | 0,5 - 2 |
| 2-Methyl-buten-1 | 7 - 11 |
| 2-Methyl-buten-2 | 15 - 20 |
| Cyclopentan | 1 - 3 |
| Diolefine/Acetylene | 0,5 - 5 |
| Gesamt-S | bis zu 1500 ppm |
| Mercaptan-S | bis zu 500 ppm |

Als Alkanole und Alkandiole für das erfindungsgemäße Verfahren seien primäre oder sekundäre, bevorzugt primäre Alkanole oder Diole mit 1-8, bevorzugt 1-4, besonders bevorzugt mit 1-2 C-Atomen genannt, wie Methanol, Ethanol, Propanol, Isopropanol, Butanol, Isobutanol, Hexandol-1, Octanol-1, Ethylenglykol, Ethylenglykol-monomethylether, Diethylenglykol, 1,2- und 1,3-Propylenglykol, 1,2-, 1,3- oder 1,4-Butylenglykol, bevorzugt Methanol, Ethanol, Ethylenglykol oder 1-2-Propylenglykol, besonders bevorzugt Methanol. Die beispielhafte Aufzählung zeigt, daß Alkanole für das erfindungsgemäße Verfahren auch Monoether von Diolen sein können und daß Alkandiole auch Diether oder Polyether von niederen Diolen sein können. In beiden Fällen ist die Alkyl- bzw. Alkylenkette durch Ether-Sauerstoff unterbrochen. Die Alkanole werden in einer Menge von 0,7-4 OH-Äquivalent/Mol Gesamtmenge tert.-Olefin im rohen Kohlenwasserstoffgemisch eingesetzt; bevorzugt ist ein Verhältnis von 0,8-2,5:1, besonders bevorzugt ein Verhältnis von 1-2:1.

Es ist bekannt, daß die Veretherung von tert.-Alkenen mit Alkanolen eine Gleichgewichtsreaktion ist, die durch einen Überschuß von OH-Äquivalenten, bezogen auf die Molmenge an tert.-Alken, zugunsten des Ethers verschoben werden kann und im Falle von i-Buten zu einer Ausbeute an Ether von bis zu 98 % der theoretischen Ausbeute führt, bei höheren tert.-Alkenen jedoch abnehmende Ausbeuten aufweist. Daher können die erfindungsgemäß erhältlichen Gemische überschüssiges C₁-C₈-Alkanol bzw. C₂-C₈-Alkandiol enthalten.

Wasserstoff wird im ertindungsgemäßen Verfahren in einer Menge eingesetzt, die mindestens äquimolar ist zu derjenigen, die nötig ist, um aus Gum-Bildnern Monoolefine zu bilden, beispielsweise im Falle eines Diolefins oder einer Acetylen-Verbindung in einer Menge von 1-2 Mol H₂/Mol Diolefin oder Acetylen-Verbindung. Wasserstoff kann in reiner oder technischer Form eingesetzt werden. Wirtschaftlich vorteilhaft kann ein in petrochemischen Anlagen anfallender, mit CH₄ und/oder N₂ vergesellschafteter Wasserstoff (Restgas) eingesetzt werden. Die H₂-Menge in solchen reinen oder technischen Wasserstoffen beträgt 70 bis 100 Vol-% H₂; in H₂-haltigen Restgasen von petrochemischen Anlagen vielfach etwa 80-90 Vol-% H₂. Bei einem geringen Arbeitsdruck von 0,5 bis 30 bar, bevorzugt 1 bis 20 bar, wird H₂ zwar aus Gründen der besseren Handhabbarkeit in größerem Überschuß angeboten, durchströmt aber zum größten Teil den Reaktor ungenutzt und wird in einem Abscheider wieder aufgefangen; dieser H₂ kann recyclisiert werden.

Das erlindungsgemäße Verfahren ist gekennzeichnet durch den Einsatz des erfindungsgemäßen katalytischen Mittels, das aus einem makroporösen oder gelförmigen, SO₃H-Gruppen enthaltenden Kationenaustauscher mit einem Vernetzungsgrad von 2-65 %, bevorzugt 8-25 % und einer spezifischen Oberfläche von 5-750 m²/g, bevorzugt 50-250 m²/g trockenen Kationenaustauschers, der mit 0,2-20 g/l Kationenaustauscher an obigen Metallen belegt ist. In bevorzugter Weise beträgt die Belegung mit den Metallen 1-20 g, besonders bevorzugt 3-15 g/l Kationenaustauscher. Bevorzugte Kombinationen sind Pd/Fe bzw. Pd/Ni. Die angegebenen Belegungsmengen werden unabhängig vom Bindungszustand als Menge des Metalls allein verstanden.

Die zugrunde liegenden makroporösen oder gelförmigen sauren Kationenaustauscher sind dem Fachmann bekannt und können beispielsweise durch Copolymerisation von Vinylmonomeren und Divinylvernetzern, gegebenenfalls in Gegenwart von Lösungsmitteln oder durch Kondensation von Phenol und Formaldehyd, hergestellt werden. Vinylmonomere sind beispielsweise Styrol oder Acrylsäureester; Divinylvernetzer ist beispielsweise Divinylbenzol. Saure Gruppen solcher Kationenaustauscher sind beispielsweise Carboxylgruppen, Phosphonsäuregruppen oder Sulfonsäuregruppen. Bevorzugt eingesetzt werden stark saure, Sulfonsäuregruppen enthaltende Styrol-Divinylbenzol-Polymerisate, die unter verschiedenen Bezeichnungen handelsüblich sind. Bevorzugt werden makroporöse Kationenaustauscher eingesetzt. Der mittlere Porenradius der makroporösen Kationenaustauscher kann beispielsweise in Grenzen von 50-1 200 Å, vorzugsweise 70-500 Å variieren. Solche Kationenaustauscher können beispielsweise als Perlpolymerisate Korngrößen von 0,1-2 mm, als Pulverharz Korngrößen von 10-100 µm, aufweisen.

Zur Belegung der Kationenaustauscher mit den genannten Metallen werden einfache oder komplexe kationische Salze der Metalle mit dem Kationenaustauscher in der H⁺-Form in an sich bekannter Weise zusammengebracht. Die Menge des aufzubringenden Metallsalzes wird berechnet oder durch einfache Vorversuche bestimmt, so daß die gewünschte Menge, gerechnet als Metall, auf dem Kationenaustauscher vorliegt.

Die Belegung mit den verschiedenen Metallen kann gleichzeitig, nacheinander oder alternierend erfolgen.

Der metalldotierte Kationenaustauscher wird neutral gewaschen, getrocknet (beispielsweise bei 80-100°C im Vakuum) und anschließend zur Überführung der aufgebrachten Edelmetalle in den elementaren Zustand mit Wasserstoff behandelt, beispielsweise bei 2-50 bar, vorzugsweise 20-30 bar und einer Temperatur von 50-140°C, vorzugsweise 80-120°C. Grundsätzlich können auch andere Reduktionsmittel, wie Hydrazin oder Formaldehyd, eingesetzt werden. Die Form, in der das zweite Metall nach der Wasserstoffbehandlung vorliegt, hängt von der Art des Metalles und von der Art der Reduktion ab. Mindestens die mit dem Edelmetall belegten Sulfonatgruppen werden durch die Wasserstoffbehandlung wieder in saure SO₃H-Gruppen umgewandelt. Die Behandlung mit Wasserstoff kann auch in Form einer Aktiviervng im Reaktor erfolgen, bevor der aktivierte = reduzierte Katalysator mit dem Kohlenwasserstoffgemisch beschickt wird.

Das erfindungsgemäße Verfahren wird bei einer Temperatur im Bereich von 30-140°C, bevorzugt 35-110°C, besonders bevorzugt 40-90°C, durchgeführt. Hierbei wird ein Druck eingestellt, bei dem das Reaktionsgemisch mit Ausnahme des nicht gelösten H₂ mindestens teilweise flüssig ist. Der Zusammenhang zwischen der gewählten Arbeitstemperatur und einem solchen einzustellenden Druck ist dem Fachmann geläufig. Im erfindungsgemäßen Verfahren wird eine LHSV (Liquid Hourly Space Velocity) von 0,1-100, bevorzugt 0,3-20, besonders bevorzugt 0,5-5 l Reaktionsgemisch (roher Kohlenwasserstoff im Gemisch mit Alkanol und Wasserstoff) pro Liter Katalysator pro Stunde eingestellt. Das katalytische Mittel aus dem Kationenaustauscher mit den beiden Metallen liegt hierbei im Festbett oder im Schwebebett vor.

Zur Durchführung des erfindungsgemäßen Verfahrens werden im allgemeinen einfache und wenig kostenintensive Apparate eingesetzt. So kann man die Einsatzstoffe, nämlich das rohe Kohlenwasserstoffgemisch, das Alkanol und H₂ getrennt oder gemeinsam durch einen Vorwärmer einem Reaktor mit dem katalytischen Mittel zuführen. Nach dem Verlassen des Reaktors mit dem katalytischen Mittel wird das Umsetzungsgemisch in einen Stabilisierungsreaktor geführt und dort von Gas, beispielsweise von überschüssigem H₂ oder gegebenenfalls vorhandenem CH₄ oder N₂ über Kopf befreit. Das dem Stabilisierungsreaktor entzogene Reaktionsgemisch liegt damit bereits in fertiger Form vor. Dieses Gemisch kann in seine Komponenten getrennt werden, beispielsweise, wenn die darin enthaltenen Alkyltert.-alkyl-ether als Lösungsmittel gewünscht werden oder beispielsweise, wenn die isolierten Ether einer Spaltung zur Gewinnung des reinen i-Alkens unterworfen werden sollen. Im Anschluß an die Abtrennung des Ethers kann weiterhin die Gewinnung der Olefine erfolgen. Diese Olefine zeichnen sich durch eine Isomerisierung aus, die etwa im Sinne einer anschließenden Herstellung von Alkylatbenzin günstig ist.

Die erfindungsgemäßen Gemische mit einem Gehalt an Alkyl-tert.-alkyl-ether sind durch eine gegenüber dem rohen Kohlenwasserstoffgemisch erhöhte Octanzahl ausgezeichnet. So enthält ein erfindungsgemäß erhältliches Kohlenwasserstoffgemisch aus einem C₅-Schnitt, wenn die Umsetzung mit Methanol vorgenommen wurde, tert.-Amyl-methyl-ether (TAME). Der Umfang der Octanzahl-Erhöhung durch den Gehalt an TAME ist selbstverständlich in einer dem Fachmann geläufigen Weise von der Menge der ursprünglich vorhandenen bzw. durch Isomerisierung hinzugewonnenen und danach veretherten i-Amylene abhängig. In vorteilhafter Weise zeigen die erfindungsgemäß erhaltenen Gemische auch eine Verringerung der Sensitivity, d.h. eine Verringerung des Abstandes zwischen der Motor-Octanzahl (MOZ) und der Research-Octanzahl (ROZ) und infolge der Eliminierung der Gum-Bildner eine Verbesserung der Farbzahl. Beispielsweise wird bei einem Gehalt an veretherbaren Alkenen im rohen Kohlenwasserstoffgemisch von beispielsweise 10-30 Gew.-% ein Gehalt an Alkyl-tert.-alkylether in den erfindungsgemäßen Verfahrensprodukten von etwa 12-42 Gew.-% erreicht.

Die Sensitivity sinkt von etwa 18-20 auf etwa 13-15, und die Farbzahl nach APHA ist stets unter 8, vielfach unter 5 und kann Werte unter 4 erreichen.

Insbesondere enthalten die ertindungsgemäß erhaltenen Gemische nur wenig Gum-Bildner, so daß die sichere Einhaltung und Unterschreitung der in DIN 51 607 und DIN 51 600 bzw. in der ASTM-Norm für Automotive Gasoline festgelegten Höchstmenge für Gum (als Abdampfrückstand) von 5 mg/100 ml Kraftstoff gewährleistet ist.

Die erfindungsgemäßen katalytischen Mittel zeigen eine hohe Standzeit auf, die durch den Gehalt an Schwefel im rohen Kohlenwasserstoffgemisch nur wenig beeinflußt wird.

### Beispiel 1

### Herstellung eines mit Ni und Pd beladenen Katalysators

2,5 l eines stark sauren, makroporösen Styrol-Divinylbenzol-Harzes mit SO₃H-Gruppen (Grundharz; Bayer-Katalysator K 2631) wurden bei Raumtemperatur in 2 l vollentsalztem Wasser gerührt. In die gerührte Dispersion wurden 3,18 g NiO gegeben. Unter weiterem Rühren wurden 194 g Salpetersäure 65 %ig zugesetzt. Es wurde 3 Stunden gerührt. Dann wurde mit vollentsalztem Wasser säurefrei gewaschen.

Der mit Ni beladene Katalysator wurde in 2 l vollentsalztem Wasser dispergiert. Innerhalb von 30 Minuten wurden 22,7 g Palladiumnitrat-Lösung (Pd-Gehalt 11 Gew.-%) zugesetzt. Es wurde 15 Minuten gerührt. Der Katalysator wurde mit vollentsalztem Wasser nitratfrei gewaschen.

### Beispiel 2

### Herstellung eines mit Ag und Pd beladenen Katalysators

2,5 l eines stark sauren, makroporösen Styrol-Divinylbenzol-Harzes mit SO₃H-Gruppen (Grundharz; Bayer-Katalysator K 2631) wurden bei Raumtemperatur in 2 l vollentsalztem Wasser gerührt. In die gerührte Dispersion wurden 2,69 g Ag₂O gegeben. Unter weiterem Rühren wurden 194 g Salpetersäure 65 %ig zugesetzt. Es wurde 3 Stunden gerührt. Dann wurde mit vollentsalztem Wasser säurefrei gewaschen.

Der mit Ag beladene Katalysator wurde in 2 l vollentsalztem Wasser dispergiert. Innerhalb von 30 Minuten wurden 22,7 g Palladiumnitrat-Lösung (Pd-Gehalt 11 Gew.-%) zugesetzt. Es wurde 15 Minuten gerührt. Der Katalysator wurde mit vollentsalztem Wasser nitratfrei gewaschen.

### Beispiel 3

### Herstellung eines mit Fe und Pd beladenen Katalysators

2,5 l eines stark sauren, makroporösen Styrol-Divinylbenzol-Harzes mit SO₃H-Gruppen (Grundharz; Bayer-Katalysator K 2631) wurden bei Raumtemperatur in 2 l vollentsalztem Wasser gerührt. In die gerührte Dispersion wurden 18,08 g Fe(NO₃)₃ * 9H₂O gegeben. Es wurde 3 Stunden gerührt. Dann wurde mit vollentsalztem Wasser säurefrei gewaschen.

Der mit Fe beladene Katalysator wurde in 2 l vollentsalztem Wasser dispergiert. Innerhalb von 30 Minuten wurden 22,7 g Palladiumnitrat-Lösung (Pd-Gehalt 11 Gew.-%) zugesetzt. Es wurde 15 Minuten gerührt. Der Katalysator wurde mit vollentsalztem Wasser nitratfrei gewaschen.

### Beispiel 4

### Herstellung eines mit Cu und Pd beladenen Katalysators

2,5 l eines stark sauren, makroporösen Styrol-Divinylbenzol-Harzes mit SO₃H-Gruppen (Grundharz; Bayer-Katalysator K 2631) wurden bei Raumtemperatur in 2 l vollentsalztem Wasser gerührt. In die gerührte Dispersion wurden 3,13 CuO gegeben. Unter weiterem Rühren wurden 194 g Salpetersäure 65 %ig zugesetzt. Es wurde 3 Stunden gerührt. Dann wurde mit vollentsalztem Wasser säurefrei gewaschen.

Der mit Cu beladene Katalysator wurde in 2 l vollentsalztem Wasser dispergiert. Innerhalb von 30 Minuten wurden 22,7 g Palladiumnitrat-Lösung (Pd-Gehalt 11 Gew.-%) zugesetzt. Es wurde 15 Minuten gerührt. Der Katalysator wurde mit vollentsalztem Wasser nitratfrei gewaschen.

### Vergleichsbeispiele 5-7, 9, 11, 12; Beispiele 8, 10

### Verfahren 1:

Ein nach Beispiel 1-4 hergestellter Katalysator wurde in einer Labor-Durchlaufapparatur, bestehend aus Vorwärmer, temperierbarem Doppelmantelreaktor und Abscheider in nachfolgend beschriebener Weise eingesetzt: 100 ml Katalysator wurden 24 Stunden in Methanol gequollen und in den Durchlaufreaktor mit 25 mm lichter Weite und Temperaturmeßstellen im Abstand von 100 mm eingefüllt. Die Aktivierung erfolgte in der Weise, daß nach einer Inertisierung mit Stickstoff bei 100°C Wasserstoff mit 30 l/h zunächst 7 Stunden bei Normaldruck, dann 7 Stunden bei 15 bar durchgeleitet wurde. Danach wurde das Einsatzprodukt eingespeist.

Als rohes Kohlenwasserstoffgemisch wurde der C₅-Strom eines Steam-Krackers (sogenannter Aromatenvorlauf) mit einem Gehalt von 11,6 Gew.-% an 2-Methylbuten-2, 3,6 Gew.-% 2-Methylbuten-1 (tertiäre Olefine) und 1,1 Gew.-% 3-Methylbuten-1 (nichttertiäres Olefin) eingesetzt. Das Einsatzprodukt enthielt neben C₅-Kohlenwasserstoffen zusätzlich einen Anteil von ca. 21 % an C₆-Kohlenwasserstoffen. Der Siedebereich dieses Einsatzmaterials liegt zwischen 35°C und 70°C, die Dichte zwischen 0,67 g/ml und 0,69 g/ml. Dieses Kohlenwasserstoffgemisch wurde mit der 1,3fachen stöchiometrischen Menge an Methanol (bezogen auf veretherbare Olefine) vermischt.

Die Belastung des Katalysators wurde mit 150 ml/h Einsatz auf eine LHSV = 1, der Druck auf 15 bar und die Temperatur des Doppelmantelreaktors auf 63°C eingestellt. Die zugegebene Menge Wasserstoff wurde so eingestellt, daß eine Abgasmenge von 10 bis 15 l/h eingehalten wurde. Nach 7 Stunden Laufzeit wurde eine Probe gezogen und das Reaktionsprodukt gaschromatographisch analysiert.

Die ermittelten Werte für den Umsatz zu TAME (t-Amyl-methyl-ether) die Hydrierung der Diene und die Isomerisierung von 3-Methylbuten-1 sind in Tabelle 1 zusammengestellt.

### Verfahren 2:

Der Versuch wurde wie in Verfahren 1 durchgeführt, jedoch wurde dem Einsatzprodukt neben Methanol zusätzlich 1 200 ppm iso-Propylmercaptan (^ 500 ppm S, bezogen auf das rohe Kohlenwasserstoffgemisch) zugemischt.

### Verfahren 3:

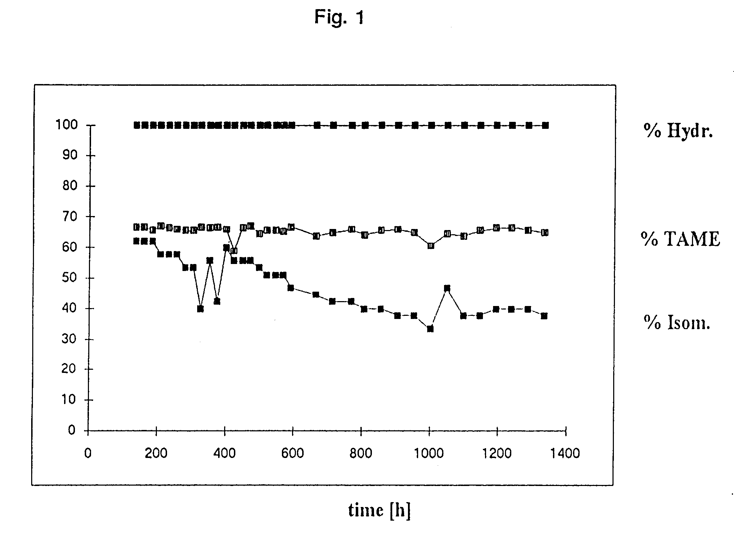

Der Versuch wurde wie in Verfahren 1 durchgeführt, jedoch wurde Katalysator nach Beispiel 1 (1 g Ni(II) + 1 g Pd(II)) verwendet und dem Einsatzprodukt 120 ppm iso-Propylmercaptan (^ 50 ppm S, bezogen auf das Kohlenwasserstoffgemisch) zugemischt. Die Umsätze zu TAME, die Hydrierung der Diene und die Isomerisierung von 3-Methylbuten-1 in Abhängigkeit von der Laufzeit ist in Fig. 1 dargestellt. Man erkennt, daß auch nach nahezu 1 400 Betriebsstunden die Hydrieraktivität für hochungesättigte Bestandteile bei 100 % liegt, daß die Veretherung annähernd gleichbleibend bei etwa 65 %, bezogen auf vorhandenes t-Amylen, liegt und der Prozentsatz der Isomerisierung sich langfristig auf etwa 40 % einstellt und dort verbleibt.

**Tabelle 1**

| Beispiel | Katalysator | Verfahren | ppm S | % TAME | % Hydr. | % Isom. |
|---|---|---|---|---|---|---|
| 5 | Grundharz | 1 | 0 | 65 | 100 | 44 |
| 6 | | 2 | 500 | 65 | 95 | 18 |
| 7 | nach Beispiel 1 | 1 | 0 | 65 | 100 | 44 |
| 8 | | 2 | 500 | 65 | 97 | 35 |
| 9 | nach Beispiel 3 | 1 | 0 | 65 | 100 | 45 |
| 10 | | 2 | 500 | 65 | 100 | 32 |
| 11 | 5 g Pd(II) allein | 1 | 0 | 65 | 100 | 45 |
| 12 | | 2 | 500 | 65 | 100 | 46 |

## Patentansprüche

1. Verfahren zur Herstellung eines Gemisches von Alkanen und Alkenen mit 4-8 C-Atomen und C₁-C₈-Alkyl-tert.-C₄-C₈-alkyl-ethern oder C₂-C₈-Alkylen-bis(C₄-C₈-tert.-alkyl-ethern), das weitgehend frei ist von hoch ungesättigten Verbindungen aus der Gruppe der Diolefine und Acetylene und das überschüssiges C₁-C₈-Alkanol oder C₂-C₈-Alkandiol enthalten kann, durch Umsetzung von
a) einem rohen Kohlenwasserstoffgemisch im Bereich von 4-8 C-Atomen, das C₄-C₈-tert.-Alkene neben anderen Alkenen und Alkanen enthält und hoch ungesättigte Verbindungen enthält, mit
b) einem C₁-C₈-Alkanol oder einem C₂-C₈-Alkandiol in einer Menge von 0,7-4, bevorzugt 0,8-2,5, besonders bevorzugt 1-2 OH-Äquivalenten, bezogen auf die Molmenge an C₄-C₈-tert.-Alken, und gleichzeitig mit
c) Wasserstoff in einer Menge von 80-500, bevorzugt 100-200, % der zur hydrierenden Umwandlung der hoch ungesättigten Verbindungen in Alkene benötigten Menge
an einem makroporösen oder gelförmigen sauren, SO₃H-Gruppen enthaltenden Kationenaustauscher mit einem Vernetzungsgrad von 2-65 % und einer spezifischen Oberfläche von 5-750 m²/g trockenen Kationenaustauschers, der mit Metallen belegt worden ist, als katalytisches Mittel, wobei gleichzeitig die Veretherung der tert.-Alkene mit den Alkanolen, die Umwandlung der hoch ungesättigten Verbindungen aus der Gruppe der Diolefine und Acetylene und eine teilweise Isomerisierung der Alkene durchgeführt werden, dadurch gekennzeichnet, daß ein rohes Kohlenwasserstoffgemisch mit einem gesamten Schwefelgehalt von 10-1500 ppm, von dem 5-500 ppm in Form von Mercaptanen, Disulfiden oder einem Gemisch beider vorliegen, eingesetzt wird und der Kationenaustauscher einen Gehalt von 0,2-20 g/l des Kationenaustauschers, gerechnet als Metall, an einem Gemisch von einem Metall aus einer ersten Gruppe von Pd, Ru, Rh und Pt mit einem Metall aus einer zweiten Gruppe von Fe, Co, Ni, Cu, Ag und W aufweist, wobei die Metalle der ersten und zweiten Gruppe im Gewichtsverhältnis von 1:4-4:1, bevorzugt 1:2-2:1, zueinander stehen.

2. Katalytisches Mittel für die verethernde, hydrierende und isomerisierende Umsetzung von rohen C₄-C₈-Kohlenwasserstoffgemischen mit einem Gehalt von 10-1500 ppm Schwefel, bestehend aus einem makroporösen oder gelförmigen, SO₃H-Gruppen enthaltenden Kationenaustauschers mit einem Vernetzungsgrad von 2-65 % und einer spezifischen Oberfläche von 5-750 m²/g trockenen Kationenaustauschers, der mit 0,2-20 g/l Kationenaustauscher, gerechnet als Metall, an einem Gemisch von einem Metall aus einer ersten Gruppe von Pd, Ru, Rh und Pt, bevorzugt Pd, mit einem Metall aus einer zweiten Gruppe von Fe, Co, Ni, Cu, Ag und W, bevorzugt Fe und Ni, belegt ist, wobei die Metalle der ersten und zweiten Gruppe im Gewichtsverhältnis von 1:4-4:1, bevorzugt 1:2-2:1, zueinander stehen.

3. Verfahren nach Anspruch 1, dadurch gekennzeichnet, daß bei einer Temperatur im Bereich von 30-140°C, bevorzugt 35-110°C, besonders bevorzugt 40-90°C, gearbeitet wird.

4. Verfahren nach Anspruch 1, dadurch gekennzeichnet, daß bei einer LHSV von 0,1-100, bevorzugt 0,3-20, besonders bevorzugt 0,5-5, gearbeitet wird.

5. Verfahren nach Anspruch 1, dadurch gekennzeichnet, daß das rohe Kohlenwasserstoffgemisch einen Gesamtschwefelgehalt von 20-1000 ppm, bevorzugt 30-500 ppm hat, von dem 10-400 ppm, bevorzugt 15-200 ppm, in Form von Mercaptanen, Disulfiden oder einem Gemisch beider vorliegen.

6. Verfahren nach Anspruch 1, dadurch gekennzeichnet, daß ein rohes Kohlenwasserstoffgemisch aus einem Fluid Catalytic Cracker (FCC) eingesetzt wird.

7. Mittel nach Anspruch 2, dadurch gekennzeichnet, daß der Kationenaustauscher ein Styrol-Divinyl-Benzol-Harz mit Sulfonsäuregruppen ist.

8. Mittel nach Anspruch 2, dadurch gekennzeichnet, daß die Metalle in einer Menge von 1-20 g, bevorzugt 3-15 g/l Kationenaustauscher vorliegen.

9. Mittel nach Anspruch 2, dadurch gekennzeichnet, daß als Metalle Pd/Fe oder Pd/Ni vorliegen.

## Claims

1. Process for the preparation of a mixture of alkanes and alkenes having 4-8 C atoms and C₁-C₈-alkyl-tert-C₄-C₈-alkyl ethers or C₂-C₈-alkylene bis(C₄-C₈-tert-alkyl ethers) which is largely free from highly unsaturated compounds from the group consisting of the diolefins and acetylenes and which can comprise excess C₁-C₈-alkanol or C₂-C₈-alkanediol by reaction of
a) a crude hydrocarbon mixture in the range of 4-8 C atoms, which comprises C₄-C₈-tert-alkenes, in addition to other alkenes and alkanes, and comprises highly unsaturated compounds, with
b) a C₁-C₈-alkanol or a C₂-C₈-alkanediol in an amount of 0.7-4, preferably 0.8-2.5, particularly preferably 1-2 OH equivalents, based on the molar amount of C₄-C₈-tert-alkene, and simultaneously with
c) hydrogen in an amount of 80-500, preferably 100-200 % of the amount required for hydrogenating conversion of the highly unsaturated compounds into alkenes
over a macroporous or gel-like acid cation exchanger which comprises SO₃H groups, has a degree of crosslinking of 2-65 % and a specific surface area of 5-750 m²/g of dry cation exchanger and has been charged with metals, as the catalytic agent, the etherification of the tert-alkenes with the alkanols, the conversion of the highly unsaturated compounds from the group consisting of the diolefins and acetylenes and partial isomerization of the alkenes being carried out simultaneously, characterized in that a crude hydrocarbon mixture having a total sulphur content of 10-1500 ppm, of which 5-500 ppm are present in the form of mercaptans, disulphides or a mixture of the two, is employed and the cation exchanger has a content of 0.2-20 g/l of the cation exchanger, calculated as metal, of a mixture of a metal from a first group of Pd, Ru, Rh and Pt with a metal from a second group of Fe, Co, Ni, Cu, Ag and W, and the metals of the first and second group are in a weight ratio of 1:4-4:1, preferably 1:2-2:1, relative to one another.

2. Catalytic agent for etherifying, hydrogenating and isomerizing reaction of crude C₄-C₈-hydrocarbon mixtures of a content of 10-1500 ppm of sulphur, comprising a macroporous or gel-like cation exchanger which comprises SO₃H groups, has a degree of crosslinking of 2-65 % and a specific surface area of 5-750 m²/g of dry cation exchanger and has been charged with 0.2-20 g/l of cation exchanger, calculated as metal, of a mixture of a metal from a first group of Pd, Ru, Rh and Pt, preferably Pd, with a metal from a second group of Fe, Co, Ni, Cu, Ag and W, preferably Fe and Ni, and the metals of the first and second group are in a weight ratio of 1:4-4:1, preferably 1:2-2:1, relative to one another.

3. Process according to Claim 1, characterized in that it is carried out at a temperature in the range of 30-140°C, preferably 35-110°C, particularly preferably 40-90°C.

4. Process according to Claim 1, characterized in that it is carried out at an LHSV of 0.1-100, preferably 0.3-20, particularly preferably 0.5-5.

5. Process according to Claim 1, characterized in that the crude hydrocarbon mixture has a total sulphur content of 20-1000 ppm, preferably 30-500 ppm, of which 10-400 ppm, preferably 15-200 ppm, are present in the form of mercaptans, disulphides or a mixture of the two.

6. Process according to Claim 1, characterized in that a crude hydrocarbon mixture from a fluid catalytic cracker (FCC) is employed.

7. Agent according to Claim 2, characterized in that the cation exchanger is a styrene/divinylbenzene resin having sulphonic acid groups.

8. Agent according to Claim 2, characterized in that the metals are present in an amount of 1-20 g, preferably 3-15 g/l of cation exchanger.

9. Agent according to Claim 2, characterized in that Pd/Fe or Pd/Ni are present as the metals.

## Revendications

1. Procédé pour la préparation d'un mélange d'alcanes et d'alcènes contenant de 4 à 8 atomes de carbone et d'alkyl(en C₁-C₈)-tert.-alkyl(en C₄-C₈)éthers ou d'alkylène(en C₂-C₈)-bis-tert.-alkyl(en C₄-C₈)éthers, qui est essentiellement exempt de composés fortement insaturés choisis parmi le groupe des dioléfines et des acétylènes, et qui peut contenir un alcanol en C₁-C₈ ou un alcane(en C₂-C₈)diol en excès, par mise en réaction
a) d'un mélange brut d'hydrocarbures dans le domaine de 4 à 8 atomes de carbone, qui contient des tert.-alcènes en C₄-C₈ en plus d'autres alcènes et alcanes, et qui contient des composés à insaturation élevée, avec
b) un alcanol en C₁-C₈ ou un alcane(en C₂-C₈)thiol en une quantité de 0,7-4, de préférence de 0,8-2,5, de manière particulièrement préférée de 1-2 équivalents OH rapportés à la quantité molaire des tert.-alcènes en C₄-C₈, et simultanément avec
c) de l'hydrogène à concurrence de 80-500, de préférence de 100-200% de la quantité requise pour la conversion à effet d'hydrogénation des composés à insaturation élevée en alcènes,
sur un échangeur de cations macroporeux ou gélatineux de type acide contenant des groupes SO₃H, possédant un degré de réticulation de 2-65% et une surface spécifique de 5-750 m²/g d'échangeur de cations à l'état sec, qui a été recouvert par des métaux, à titre d'agent catalytique, dans lequel l'éthérification des tert.-alcènes avec les alcanols, la conversion des composés à insaturation élevée choisis parmi le groupe des dioléfines et des acétylènes et une isomérisation partielle des alcènes sont réalisées simultanément, caractérisé en ce qu'on met en oeuvre un mélange brut d'hydrocarbures possédant une teneur totale en soufre de 10-1500 ppm, dont 5 à 500 ppm sont présents sous forme de mercaptans, de disulfures ou d'un mélange des deux, et l'échangeur de cations présente une teneur, à concurrence de 0,2-20 g/l d'échangeur de cations, calculés comme métal, en un mélange d'un métal d'un premier groupe comprenant Pd, Ru, Rh et Pt avec un métal d'un second groupe comprenant Fe, Co, Ni, Cu, Ag et W, le rapport pondéral des métaux des premier et deuxième groupes s'élevant à 1:4-4:1, de préférence à 1:2-2:1.

2. Agent catalytique pour la mise en réaction à effet d'éthérification, d'hydrogénation et d'isomérisation de mélanges bruts d'hydrocarbures en C₄-C₈ possédant une teneur en soufre de 10-1500 ppm, constitué par un échangeur de cations macroporeux ou gélatineux contenant des groupes SO₃H, possédant un degré de réticulation de 2-65% et une surface spécifique de 5-750 m²/g d'échangeur de cations à l'état sec, qui est recouvert, à concurrence de 0,2-20 g/l de l'échangeur de cations, calculés comme métal, avec un mélange d'un métal choisi parmi un premier groupe comprenant Pd, Ru, Rh et Pt, de préférence Pd, avec un métal choisi parmi un deuxième groupe comprenant Fe, Co, Ni, Cu, Ag et W, de préférence Fe et Ni, le rapport pondéral des métaux des premier et deuxième groupes s'élevant à 1:4-4:1, de préférence à 1:2-2:1.

3. Procédé selon la revendication 1, caractérisé en ce qu'on travaille à une température dans le domaine de 30 à 140°C, de préférence de 35 à 110°C, de manière particulièrement préférée de 40 à 90°C.

4. Procédé selon la revendication 1, caractérisé en ce qu'on travaille à une LHSV (vitesse spatiale horaire de liquide) de 0,1-100, de préférence de 0,3-20, de manière particulièrement préférée de 0,5-5.

5. Procédé selon la revendication 1, caractérisé en ce que le mélange brut d'hydrocarbures possède une teneur totale en soufre de 20-1000 ppm, de préférence de 30-500 ppm, dont 10-400 ppm, de préférence 15-200 ppm sont présents sous forme de mercaptans, de disulfures ou d'un mélange des deux.

6. Procédé selon la revendication 1, caractérisé en ce qu'on met en oeuvre un mélange brut d'hydrocarbures provenant d'un Fluid Catalytic Cracker (FCC).

7. Agent selon la revendication 2, caractérisé en ce que l'échangeur de cations est une résine de styrène-divinyl-benzène contenant des groupes d'acide sulfonique.

8. Agent selon la revendication 2, caractérisé en ce que les métaux sont présents en une quantité de 1-20 g, de préférence de 3-15 g/l d'échangeur de cations.

9. Agent selon la revendication 2, caractérisé en ce que sont présents comme métaux, Pd/Fe ou Pd/Ni.
